# EUROPEAN PATENT SPECIFICATION

(11) **EP 2 421 460 B1**
(45) Date of publication and mention of the grant of the patent: **16.10.2013**
(21) Application number: 10767824.5
(22) Date of filing: 23.04.2010
(51) Int. Cl.: A61B 18/02, A61B 17/32, A61B 17/3211, A61H 39/06, A61B 18/08

(54) **APPARATUSES FOR APPLYING A CRYOGENIC EFFECT TO TISSUE AND CUTTING TISSUE**
VORRICHTUNGEN ZUR ANWENDUNG EINER KRYOGENEN WIRKUNG AUF GEWEBE UND ZUM SCHNEIDEN VON GEWEBE
APPAREILS POUR APPLIQUER UN EFFET CRYOGÉNIQUE À UN TISSU ET POUR COUPER LE TISSU

(30) Priority: 23.04.2009 US 428925
(43) Date of publication of application: 29.02.2012
(73) Proprietor: CSA Medical, Inc., Baltimore, MD 21218 (US)
(72) Inventor: KRIMSKY, William, S., Bel Air MD 21015 (US)
(74) Representative: Brophy, David Timothy
(86) International application number: PCT/US2010/032192
(87) International publication number: WO 2010/124178

(56) References cited:
- WO-A1-98/52479
- US-A- 3 786 814
- US-A- 4 770 171
- US-A- 5 211 646
- US-A1- 2001 023 348
- US-B1- 6 270 493
- US-B2- 7 381 208

## Description

### BACKGROUND OF THE INVENTION

The application of cryogen has been used in medicine to freeze healthy or diseased tissue. Typically, a lesion or other undesired tissue is frozen using either the direct application of a cryogen or by contacting the tissue with a cryogenic probe. After freezing, the treated tissue will generally become necrotic and will slough off allowing the growth of new tissue to replace the treated tissue.

Cryotherapy has been used in combination with other surgical techniques. An example of such a combination is provided by the cryo-scalpel disclosed in United States Patent No. 3,662,755. In this device, a refrigerant is injected into the blade of the scalpel in order to reduce the temperature of the scalpel. The scalpel is also equipped with a heater that permits heating of the blade to permit detachment from the frozen tissue. Since cooling of the target tissue is effected by contacting the tissue with the cooled blade, this limits the speed at which tissue is cooled. In addition, since the blade is at a temperature below the freezing point of water, the blade will tend to freeze to the tissue being cut.

There remains a need in the art for apparatuses and methods for cooling tissue that will be cut that overcomes the difficulties of the prior art. This need and others is met by the apparatuses and methods disclosed herein.

### SUMMARY OF THE INVENTION

The disclosure relates to surgical devices adapted to administer cryogen to a target tissue and cut tissue. The surgical devices may comprise a blade attached to a handle; wherein the handle and/or the blade include one or more cryogen delivery orifices. Typically, the orifice(s) is adapted to spray cryogen to a tissue in proximity to the blade or to create an isotherm in proximity to the blade. Any suitable cryogen may be used in conjunction with the surgical devices disclosed herein. An example of a suitable cryogen is a low temperature gas, for example, a liquefied gas. Examples of suitable cryogens include, but are not limited to, oxygen, nitrogen, argon, carbon dioxide, and air.

In some embodiments, a surgical device may be equipped with one or more blades wherein at least one blade is removably attached to the handle. According to some embodiments, the handle includes one or more cryogen delivery orifices. In some surgical devices of the invention, the blade includes one or more cryogen delivery orifices. According to further embodiments, the blade and the handle each define one or more cryogen delivery orifices.

In addition to being adapted to administer cryogen, a surgical device may also be adapted to administer heat to the device itself and/or to a target tissue which may be the same or different tissue as the tissue to which cryogen is administered. In some embodiments, a surgical device may include one or more heating elements. The heating element(s) may be adapted to heat any portion of the device. According to some the heating element(s) may be adapted to heat the blade. According to some embodiments, the heating element(s) may be adapted to heat the handle. According to further embodiments, the heating element(s) may be adapted to heat both the blade and the handle.

According to some embodiments, a surgical device may comprise one or more cooling elements. The cooling element(s) may be disposed at any position in the surgical device. In some embodiments, the cooling element(s) is adapted to use Joule-Thompson cooling. In some embodiments, the cooling element(s) may be adapted to cool the blade of the surgical device. In other embodiments, the cooling element(s) may be adapted to cool the handle. According to further embodiments, the cooling element(s) may be adapted to cool both the blade and the handle.

According to some embodiments, a surgical device may comprise one or more heating elements and one or more cooling elements.

According to some embodiments, a surgical device may comprise a cryogen supply tube attached to the device. The cryogen supply tube may be attached to any portion of the device, for example, the handle and/or the blade. Typically, the cryogen delivery orifice(s) is adapted to receive cryogen from the cryogen supply tube, and spray cryogen to a tissue or create an isotherm in proximity to the tissue. The tissue may be in proximity to the blade. Any cryogen known to those skilled in the art may be used in conjunction with a surgical device including a cryogen supply tube. An example of a suitable cryogen is a low temperature gas, for example, a liquefied gas. Examples of suitable cryogens include, but are not limited to, oxygen, nitrogen, argon, carbon dioxide, and air.

The invention is defined in the claims

### BRIEF DESCRIPTION OF THE DRAWINGS

Figure 1A is a side view of a surgical device according to an embodiment.

Figure 1B is a sectional view of the surgical device of Figure 1A.

Figure 2A is a sectional view of a surgical device according to another embodiment.

Figure 2B is a rear end view showing a handle of the surgical device of Figure 2A.

Figure 3 is a sectional view of a surgical device according to another embodiment.

Figure 4 is a perspective view of a surgical device according to another embodiment.

Figure 5 is sectional view of a handle for a surgical device according to another embodiment.

Figure 6 shows another embodiment, in which a surgical device includes a blade adapted to be heated and/or cooled during use.

Figure 7A is a partial side view of a surgical device according to another embodiment.

Figure 7B is an end view showing a blade of the device of Figure 7A.

### DETAILED DESCRIPTION OF THE INVENTION

The following description discloses various embodiments of surgical devices for applying cryogenic material ("cryogen") to tissue and cutting tissue. In the various embodiments that follow, reference characters shared among the embodiments indicate similar components and/or features. The surgical devices disclosed herein may be used in conjunction with any known system, method and/or device for the delivery of cryogen to a target tissue. Examples of suitable devices for the delivery of cryogen include, but are not limited to, those disclosed in United States patent nos. 6,027,499, 6,383,181, 7,025,762, and 7,255,693 issued to Johnston et al.

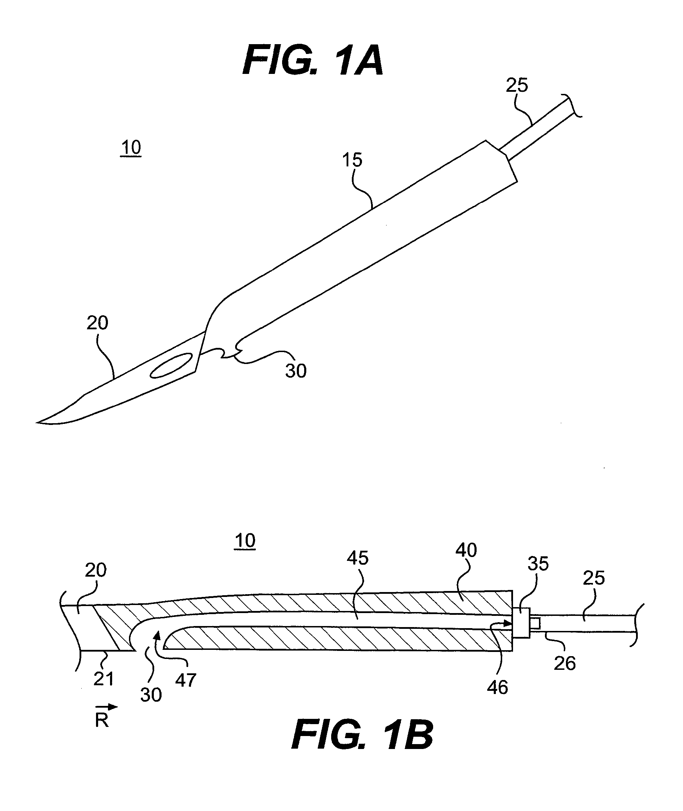

With reference to Figure 1A, a surgical device **10** is depicted. The surgical device **10** is includes a handle **15** and a blade **20.** A cryogen supply tube **25** is attached to the handle **15** and is in fluid communication with a cryogen storage device, for example, a container of liquefied gas (not shown). The handle **15** includes a cryogen delivery orifice **30** for spraying a cryogen delivered by the cryogen supply tube **25.** Referring to Figure 1B, the surgical device **10** may include a fluid channel or cryogen channel **45** for flowing a cryogen received from the cryogen supply tube **25.** The channel **45** may have a first end **46** for receiving the cryogen from the delivery tube **25** and a second end **47** terminating at the cryogen delivery orifice **30.** The channel **45** may be an elongate, open pathway, and may have any suitable cross-sectional shape. It should be understood that, although a single cryogen channel **45** and a single orifice **30** are shown, it is possible for the handle **15** to include multiple orifices **30** or multiple cryogen channels **45** each terminating at one or more orifices **30.**

The exit end **26** of the cryogen supply tube **25** is shown attached to the handle **15** via a fitting **35.** The fitting **35** may be any fitting suitable for attaching the cryogen supply tube **25** to the handle **15.** Examples of suitable fittings include, but are not limited to, a luer fitting, a threaded fitting, a compression fitting and the like. However, it should be understood that other means for attaching the cryogen supply tube **25** to the handle **15** are possible. For example, a fitting may be provided inside of the channel **45** for attaching the tube **25,** or the exit end **26** of the tube **25** may be inserted directly into the channel **45** and retained in the channel **45** by a friction fit or an adhesive bond with the walls of the channel **45.**

As shown in Figure 1B, the internal portion of handle **15** may include an insulating material **40** surrounding the cryogen channel **45.** The insulating material **40** may be the same material that is used to form remaining portions of the handle **15,** or it may be a different material. Any material that is suitable to insulate the user's hand from the cryogen delivered through the handle may be used. Suitable examples of insulating materials that may be used include, but are not limited to, polycarbonate, fluoropolymer, polyimide, polyetheretherketone (PEEK), polyetherimide/ULTEM, polyamide-imide, and combinations thereof. In some embodiments, combinations of materials may be used to fabricate the handle. In some embodiments, different materials may be used to fabricate different portions of the handle, for example, portions of the handle that will be exposed to low temperatures (e.g., portions close to the blade and or cryogen orifices) may be fabricated with one material and another material may be used to fabricate the portion of the handle contacted by the user, for example, a non-slide material may be used to fabricate all or a portion of the handle gripped by the user.

In operation of the device **10,** cryogen flows from a cryogen storage device (not shown) through the cryogen supply tube **25,** then is delivered to the cryogen channel **45** from the cryogen supply tube **25** via the fitting **35,** then flows through the cryogen channel **45,** and then exits the handle **15** through the cryogen delivery orifice **30.**

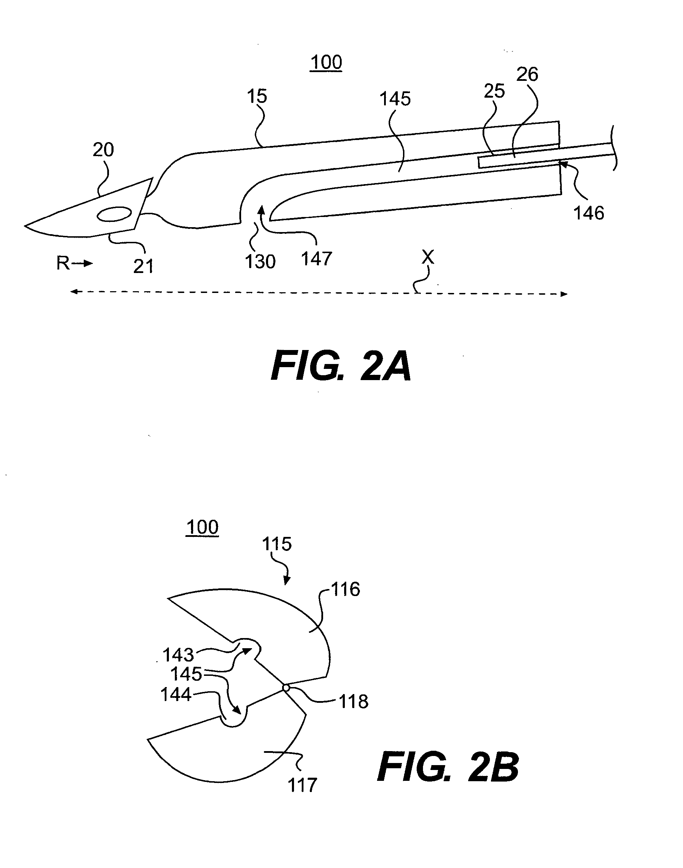

Figures 2A and 2B show a surgical tool **100** according to another embodiment. As shown in Figure 2A, the tool **100** includes a handle **115** and a blade **20** attached to the handle **115.** The handle **115** defines an interior cryogen channel **145** that is generally similar to the channel **45** described in the previous embodiment. The cryogen channel **145** has a first end **146** for receiving an exit end **26** of a cryogen supply tube **25** and a second end **147** that terminates at a cryogen delivery orifice **130.** Referring to Figure **2B****,** which shows the handle **115** in an open position for receiving the cryogen supply tube **25,** the handle **115** is formed by two mating handle portions **116, 117** that are at least partially separable along the axial direction **X** of the handle generally corresponding to the axial path of the cryogen channel **145.** As illustrated in Figure 2B, the portions **116, 117** may be pivotally attached to each other by a longitudinally extending hinge or pivot connection **118.** The pivot connection **118** may be formed of any material and may be integral to the handle **115.** Alternatively, the pivot connection **118** may be a separate part and the handle **115** may be formed of the two portions **116, 117** with the pivot connection **118** attached to both of the portions **116, 117.** In some embodiments, the pivot connection **118** may be formed of the material used to form the remainder of the handle **115.** For example, the pivot connection **118** may be a flexible portion of the material of the handle **115** that allows the handle **115** to be opened and closed. The portions **116, 117** may include respective channel segments **143, 144** that combine to form the cryogen channel **145** when the handle **115** is closed.

To attach the cryogen supply tube **25** to the handle **115,** the handle **115** can be opened by pivoting the portions **116, 117** apart about the pivot connection **118,** the cryogen supply tube **25** can then be aligned with the channel segments **143, 144,** and the handle **115** can then be closed around the cryogen supply tube **25** by pivoting the portions **116, 117** towards each other and securing the portions **116, 117** together such that the cryogen supply tube **25** is positioned in the cryogen channel **145.** The portions **116, 117** may be secured together by any suitable mechanism such as a detent mechanism with interlocking male and female latching or locking members (not shown). The cryogen supply tube **25** may be conveniently removed from the handle **115** if desired by opening the handle **115.** In this embodiment, the exit end **26** of the cryogen supply tube **25** is retained in the cryogen channel **145** by a friction fit with the walls of the channel **145.** However, according to other embodiments, the cryogen supply tube **25** may be removably or permanently affixed in position, for example, by attaching the cryogen supply tube **25** to a fitting (not shown) or adhesive placed in the cryogen channel **145.** According to additional embodiments, the pivot connection **118** may be eliminated such that the handle segments **116, 117** are completely separable from each other and can be secured together by simply pressing the segments **116, 117** together to engage one or more detent mechanisms.

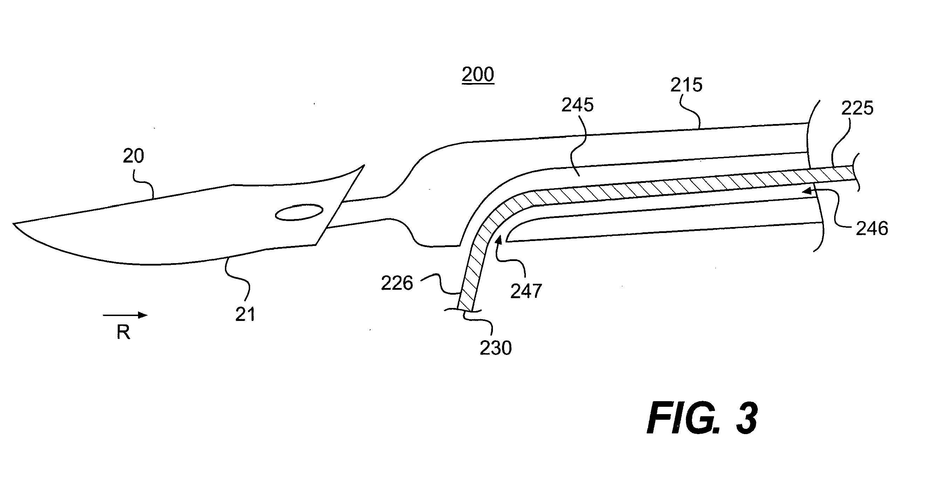

Figures 1A-1B and Figures 2A-2B depict embodiments in which the cryogen delivery orifice is formed as part of handle. However, other arrangements are envisioned. For example, a cryogen delivery orifice may be a separate part attached to the end of a cryogen supply tube or may be an opening in cryogen supply tube, as shown in FIG. 3. Referring to FIG. 3, a surgical device **200** according to another embodiment includes a handle **215** and a blade **20.** The surgical device **200** includes an elongate open channel **245** formed in an interior region of the handle **215** and a cryogen supply tube **225** extending through the channel **245.** Thus, the channel **245** retains and guides the cryogen supply tube **225.** The channel **245** includes a first end **246** of that defines an entry way through which the tube **225** can be inserted into the channel **245,** and a second end **247** that defines and exit through which an exit end **226** of the tube **225** protrudes from the handle **215.** The cryogen supply tube **225** may include one or more cryogen delivery orifices **230.** The cryogen delivery orifice **230** may be formed by an opening in the cryogen supply tube **225,** or it may be formed by a fitting or nozzle attached to the exit end **226** of the tube **225.** It should be understood that, although only one channel **245** and associated cryogen supply tube **225** are shown, it is possible to provide the handle **215** with multiple channels **245** each having a cryogen supply tube **225** therein, or a single channel **245** having multiple cryogen supply tubes **225** therein. It should also be understood that the handle **215** may optionally be formed by at least partially separable segments in a manner similar to that described with respect to the embodiment of Figures 2A and 2B.

In operation of the device **200,** cryogen is delivered through the cryogen supply tube **225,** and then exits the tube **225** through the cryogen delivery orifice **130.**

In each of the embodiments depicted in Figures 1A-3, the cryogen delivery orifice **30/130/230** is positioned at an underside of the handle **15/115/215** in order to apply cryogen to tissue below the handle, typically in the cutting path or vicinity of the cutting edge **21** of the blade **20,** which is located at a bottom surface of the blade **20.** It should be understood however, that the orifices **30, 130, 230** may be positioned at any desired location in or on the respective handles **15, 115, 215.** It should also be understood that, although the cryogen channels **45, 145, 245** are shown and described as being located in the interior space of the handles **15, 115, 215,** the cryogen channels **45, 145, 245** may be formed as tubes attached to or formed on an outer wall of the handles **15,115, 215.**

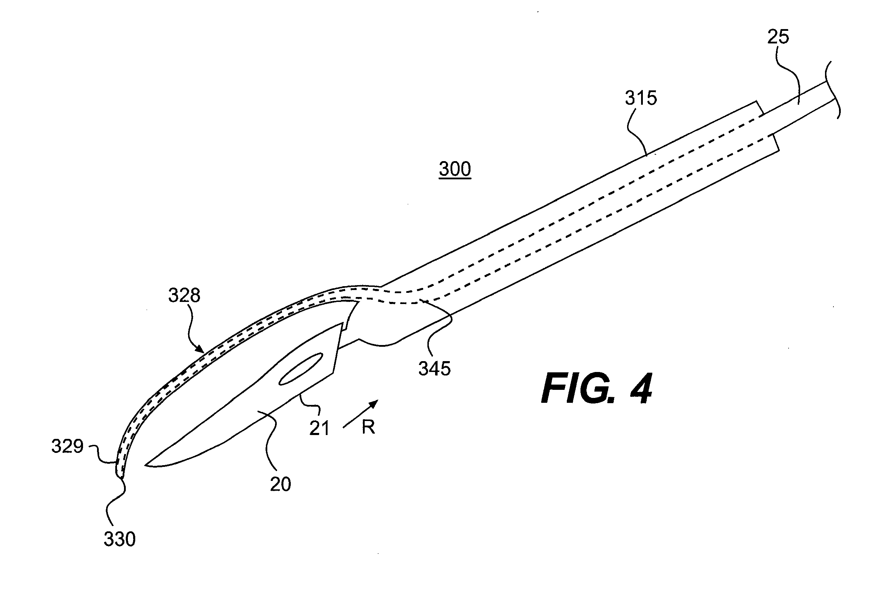

In the embodiments shown in Figures 1A-3, the surgical devices **10, 100, 200** are configured to facilitate delivering cryogen to a target tissue prior to contacting the tissue with blade **20,** due to the fact that the blade **20** is positioned in front of the cryogen delivery orifice **30/130/ 230** and the blade is typically used in a rearward cutting motion indicated by the direction **R** (Figures 1B, 2A and 3). One skilled in the art will appreciate that it may also be desirable to contact tissue during and/or after contacting the tissue with the blade. Figure 4, which is described below, shows as surgical device **300** configured to facilitate delivering cryogen to a target tissue after the tissue has been contacted with the blade **20.** Although the devices **10, 100, 200** are stated to be configured for delivering cryogen to tissue before cutting the tissue and the device **300** is stated to be configured for delivering cryogen to tissue after cutting the tissue, it should be understood that each of the devices **10, 100, 200, 300** can be used for delivering cryogen to tissue before, during or after cutting the tissue.

Referring to Figure 4, the surgical device **300** includes a handle **315** and a blade **20** attached to the handle **315.** A cryogen supply tube **25** is attached to the handle **315.** The tube **25** may be directly attached to the handle **315** by a friction fit or adhesive bond, or by an internal or external fitting as described with respect to previous embodiments. The device **300** also includes a cryogen application tube **328** attached to and extending from the handle **315** and extending forward from the handle **315** and over top of the blade **20.** The cryogen application tube **328** may be formed integrally with the handle **315,** or alternatively, may be attached to the handle **315** by a fitting (not shown), an adhesive bond, or a friction fit within an opening (not shown) in the handle **315.** The cryogen application tube **328** includes an exit end **329** and a cryogen delivery orifice **330** at the exit end **329.** A cryogen channel **345** extends through the interior of the handle **315** and the cryogen application tube **328.** Alternatively, the cryogen channel **345** may be formed as tube attached to or formed on an outer wall of the handle **315.**

The channel **345** is in fluid communication with the cryogen supply tube **25** and terminates at the orifice **330.** In operation of the device **300,** cryogen is delivered through the cryogen supply tube **25,** and then exits the tube **25** through the cryogen delivery orifice **330.**

As shown in FIG. 4, the exit end **329** of the cryogen application tube **328** may be positioned in front of the forward tip of the blade **20,** such that cryogen can be applied to tissue through the orifice **330** in proximity to the blade **20** after the tissue has been cut using a rearward **(R)** cutting motion. In this embodiment, the cryogen application tube **328** extends over top of the blade **20** so as not to interfere with the cutting edge **21** of the blade **20,** which is located at the bottom of the blade **20.** One skilled in the art will appreciate that the cryogen application tube **328** can be configured and/or positioned so as to spray cryogen onto the blade for example, in the middle of the blade **20.** The cryogen application tube **328** may optionally be formed from a deformable material, including but not limited to a flexible rubber, plastic, metal or steel material, such that the exit end **329** of the cryogen application tube **328** can be selectively placed in various positions with respect to the blade **20.** It should also be understood that the cryogen application tube **328** can be incorporated in other devices, such as the devices shown in Figures 1-3.

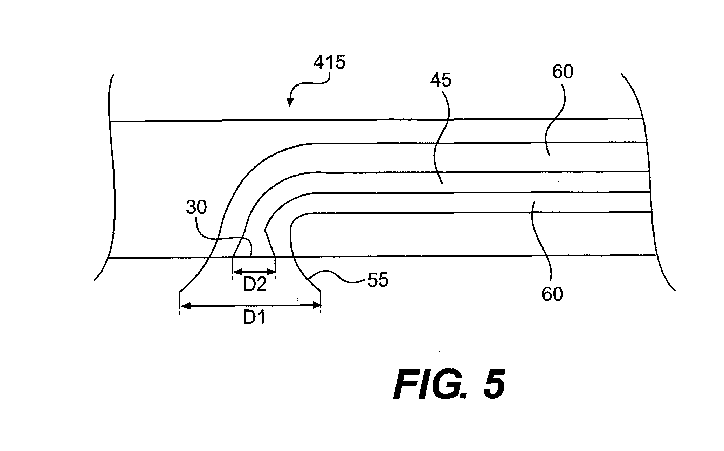

In Figure 5, a portion of a handle **415** for a surgical device similar to the devices of the previous embodiments is shown. The handle **415** is equipped with a suction hood **55.** In the embodiment shown, the suction hood **55** is disposed around a cryogen delivery orifice **30.** One skilled in the art will appreciate that suction hood **55** may be placed in other relationships to the cryogen delivery orifice **30.** For example, the cryogen delivery orifice **30** may be adjacent to the suction hood **55.** The diameter **D1** of the suction hood **55** may be of any size, i.e., it may be larger, the same size, or smaller than the diameter **D2** of the cryogen delivery orifice **30.** A surgical device may be equipped with a plurality of suction hoods that may be the same or different sizes. Typically, when a handle such as the handle is provided with a suction hood, the handle will also be provided with a suction tube or channel for applying suction in proximity to target tissue. In the embodiment shown in Figure 5, a suction tube, passage or channel **60** is shown disposed around the cryogen channel **45.** The suction channel **60** is in fluid communication with a suction pump or another conventional device (not shown) for creating suction. One skilled in the art will appreciate that the suction channel **60** may have any spatial relationship with regard to the cryogen channel **45,** e.g., may run along side of the channel **45** or may run along the outside of the handle **415.** In one embodiment, the suction channel **60** may be formed in the handle **415** and is coaxial with the channel **45.**

In operation, cryogen may be delivered through the channel **45** and may exit the cryogen delivery orifice **30.** At the same time, suction may be applied to the suction channel **60.** The handle **415** may be oriented such that the suction hood **55** is in proximity to, but not in contact with, the target tissue. As the cryogen exits the cryogen delivery orifice **30,** it may contact the target tissue or create an isotherm in proximity to the target tissue and then be drawn up into the suction hood **55** which then directs the cryogen into the suction channel **60.** One skilled in the art will understand that a suction hood and suction channel such as the suction hood 55 and suction channel **60** in the instant embodiment may employed in any of the other embodiments described herein.

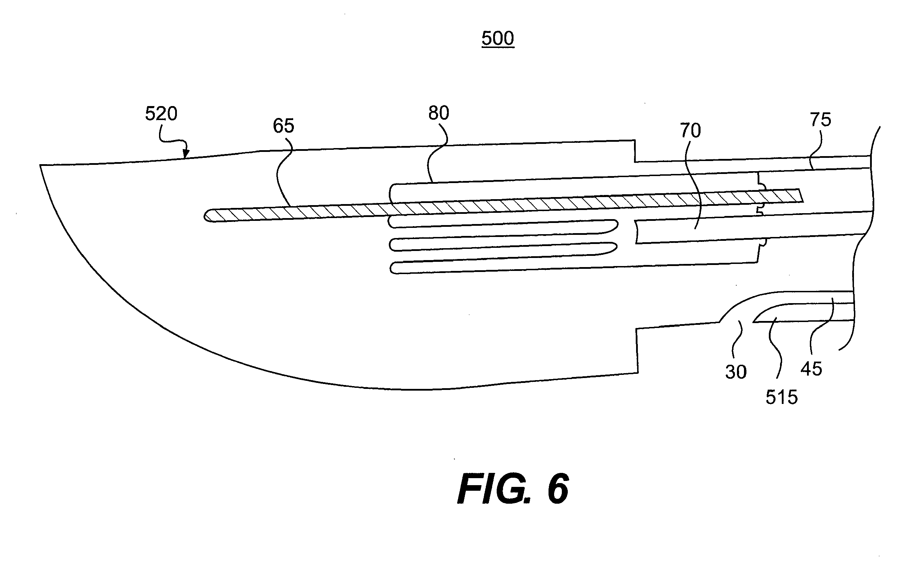

In some embodiments, a blade of a surgical device may be a heated and/or cooled blade. Figure 6 shows a partial view of a surgical device **500** including a blade **520** that can be heated and cooled, and an handle **515** attached to the blade **520.** The handle **515** includes a cryogen channel **45** and a cryogen delivery orifice **30.** The blade **520** includes an auxiliary cryogen supply tube **65** that is configured to deliver cryogen to an interior space of the blade **520.** The auxiliary cryogen supply tube **65** may be in fluid communication with the same source of cryogen used by another, primary cryogen supply tube such as the cryogen supply tubes **25, 225,** described in previous embodiments, or it may be in fluid communication with a different cryogen source. The auxiliary cryogen supply tube **65** is adapted to deliver cryogen in the form of liquefied gas. Those skilled in the art will appreciate that auxiliary cryogen supply tube **65** could be equipped with a nozzle (not shown) with a suitable aperture and cooling could be effected by forcing pressurized gas through the nozzle and utilizing the Joule-Thomson effect.

The blade **520** is also equipped with a suction tube or channel **70.** The auxiliary cryogen supply tube **65** is in fluid communication with a cryogen source, the interior space of blade **520,** and the suction channel **70.** The suction channel **70** may extend through the handle **515** and may be connected to a suction device (not shown). In use, cryogen may be delivered to the interior space of the blade **520** to effect cooling of the blade and then may exit the interior space of the blade **520** via the suction tube.

The device **500** also includes an electrical connection **75** connected to a heating element **80** disposed in the blade **520.** Electrical current can be caused to flow through the heating element **80,** resulting in heating of the blade **520.** The blade **520** may be heated and/or cooled as necessary or desirable during the course of surgery by causing electricity to flow through the heating element and/or cryogen to flow through an auxiliary cryogen supply tube **65.** It should be understood that the blade **520** could be modified, if desired, to include only heating components or cooling components, as opposed to both heating and cooling components as shown in Figure 6.

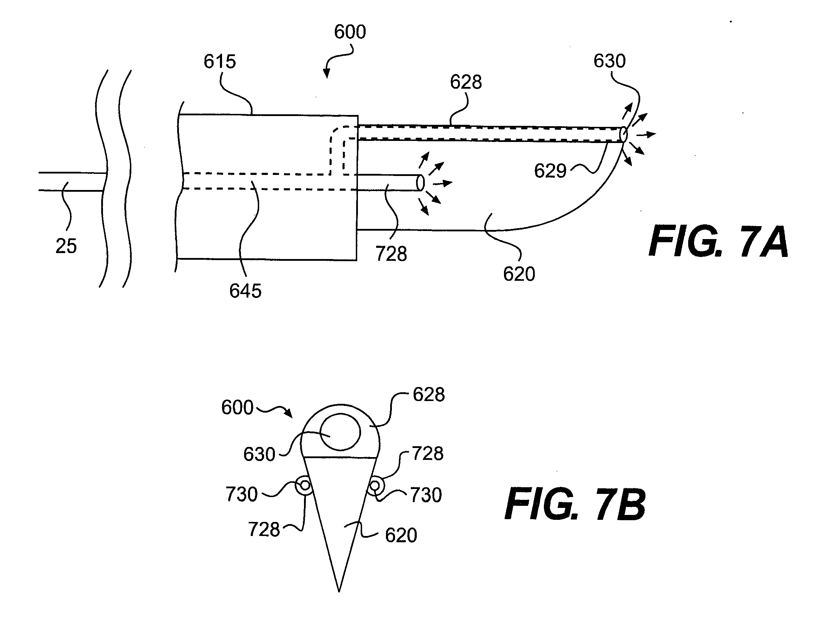

Figures 7A and 7B show surgical device **600** according to another embodiment. The device **600** is particularly configured for spraying cryogen on or in proximity to tissue while the tissue is being cut, but may also be used to spray cryogen on tissue before or after cutting the tissue. The surgical device **600** includes a handle **615** and a blade **620** attached to the handle **615.** The blade **620** may include a cryogen application tube **628** generally extending along a top edge of the blade **620,** and a pair of cryogen application tubes **728** generally extending along the side surfaces of the blade **620.** The cryogen application tubes **628, 728** include respective cryogen delivery orifices **630, 730** at exit ends **629, 729** of the tubes **628, 728.** The cryogen application tubes **628, 728** may be separate parts attached to the blade **620,** or they may be integrally formed with the blade **620.** The orifices **630, 730** may be in fluid communication with a cryogen channel **645** extending through or along the handle **615.** The cryogen channel **645** may be connected to a cryogen supply tube **25** as described with respect to preceding embodiments discussed herein. Alternatively, the cryogen channel **645** may be eliminated and one or more cryogen supply tubes similar to the illustrated tube **25** may extend within or along the handle **615** and be connected directly to the cryogen application tubes **628, 728.** The cryogen application tubes **628, 728** can both spray target tissue and cool the blade **620** due to their positioning. It is also possible to provide a blade having either the tube **628** or tubes **728,** rather than both the tube **628** and tubes **728**. One skilled in the art will also understand the blade **620** may also be used with the handles **15, 115, 215, 315, 415** disclosed in other embodiments herein.

Each of the embodiments disclosed herein is shown as having a static knife-type blade **20/520/620.** However, it is possible to provide other types of blades. For example, a motorized reciprocating blade, a serrated blade, or a rotary blade may be provided.

The surgical devices **100, 200, 300, 500, 600** described above may be connected to a cryogen storage device, such as a tank of liquefied gas, for supplying cryogen to the surgical devices. The cryogen storage device may be a component of a cryogen delivery system capable of controlling the flow of cryogen to the cryogen supply tube **25/225.** Typically, the flow of cryogen from the cryogen storage device to the surgical device **100/200/300/500/600** may be controlled using any suitable valve known to those skilled in the art. Such a valve is typically equipped with an actuator and is capable of being actuated remotely by a control device, for example, by a foot pedal and/or hand operated button. In operation, the valve is actuated to open the flow path from the cryogen storage device through the cryogen supply tube **25/225,** thereby causing cryogen to travel from the cryogen storage device through the cryogen supply tube **25/225** and exit out of the cryogen delivery orifice **30/130/230/330.**

The devices **100/200/300/500/600** disclosed herein may be used, for example, to conduct surgery by spraying cryogen on a tissue of a patient or creating an isotherm in proximity to the tissue with cryogen and contacting the tissue with the blade **20/520/620.** The tissue to be contacted with the blade **20/520/620** may be treated with cryogen at any time, for example, before being contacted with the blade, while being contacted with the blade, and/or after being contacted with the blade. When the device **500** is employed, surgery may be performed by spraying cryogen on the tissue of the patient or creating an isotherm in proximity to the tissue with cryogen, and heating the tissue prior to contacting the tissue with the blade **520,** while contacting the tissue with the blade **520,** and/or after contacting the tissue with the blade **520.** When the devices **500, 600** are employed, a tissue may be treated by spraying cryogen on the tissue or creating an isotherm in proximity to the tissue with cryogen, and further cooling the tissue prior to contacting the tissue with the blade, while contacting the tissue with the blade, and/or after contacting the tissue with the blade.

While the foregoing invention has been described in specific exemplary embodiments, it will be appreciated by one skilled in the art from a reading of this disclosure that various changes in form and detail can be made without departing from the true scope of the invention and appended claims.

## Claims

1. A surgical device, comprising: a handle; a blade attached to the handle **characterised by** at least one orifice configured to spray a cryogen from the surgical device, the at least one orifice being disposed in or on at least one of the handle and the blade.

2. The surgical device of claim 1, wherein the at least one orifice comprises an orifice positioned at one of the following locations: an underside of the handle, in front of a tip of the blade, a side surface of the blade, and a top edge of the blade.

3. The surgical device of claim 1, comprising at least one channel configured to direct the cryogen to the at least one orifice, wherein the at least one channel extends through an interior of the handle or is disposed on an outer wall of the handle.

4. The surgical device of claim 3, comprising a fluid supply tube configured to be placed in fluid communication with a source of the cryogen, wherein the at least one channel is configured to be placed in fluid communication with the fluid supply tube.

5. The surgical device of claim 3, wherein the at least one orifice is disposed in a fluid application tube attached to or disposed on the handle or the blade, and wherein the at least one channel comprises a channel extending through the fluid application tube.

6. The surgical device of claim 3, wherein the at least one orifice is disposed in a liquid application tube attached to or disposed on the handle or the blade, and wherein the at least one channel comprises a channel extending through the liquid application tube.

7. The surgical device of claim 1 or 3-6, wherein the least one orifice is adapted to be in fluid communication with a source of the cryogen, and wherein the at least one orifice is configured to spray the cryogen to a tissue in proximity to the blade or to create an isotherm in proximity to the blade.

8. The surgical device of claim 3, wherein the cryogen is a liquefied gas.

9. The surgical device of claim 3, wherein the cryogen is selected from the group consisting of oxygen, nitrogen, argon, carbon dioxide, and air.

10. The surgical device of claim 1, comprising at least one heating element configured to heat the blade.

11. The surgical device of claim 1, wherein the blade comprises at least one cooling element, optionally wherein the at least one cooling element comprises at least one tube adapted to carry a cooling fluid, and optionally wherein the cooling fluid is the cryogen.

12. The surgical device of claim 1, wherein the blade is removably attached to the handle.

13. The surgical device of claim 1 or 3, wherein the device comprises a suction passage configured to suction an excess portion of the cryogen after the cryogen is sprayed.

14. The surgical device of claim 13, wherein the suction passage is coaxial with or adjacent to the channel.

15. The surgical device of claim 13, comprising a suction hood configured to direct the excess portion of the cryogen to the suction passage.

16. The surgical device of claim 14, wherein the suction hood is disposed around the at least one orifice.

17. The surgical device of any one of claims 1, 2, 7-9 or 10-12, further comprising: a fluid supply tube attachable to the handle; wherein the at least one orifice is configured to spray the cryogen delivered through the fluid supply tube.

18. The surgical device of claim 16, comprising a channel extending through the handle or disposed on the handle, wherein the channel is configured to retain the fluid supply tube.

19. The surgical device of claim 16, wherein the fluid supply tube is configured to be placed in fluid communication with the channel, and wherein the at least one orifice is disposed at an end of the channel.

20. The surgical device of claim 16, comprising a channel extending through the handle or disposed on the handle, wherein the fluid supply tube is configured to extend through the channel such that a dispensing end of the fluid supply tube protrudes from the handle through an end of the channel, and wherein the at least one orifice comprises an orifice disposed at the dispensing end of the fluid supply tube.

21. The surgical device of claim 16, wherein the handle comprises a first handle portion and a second handle portion, wherein the first and second handle portions are at least partially separable from each other along an axial direction of the handle for inserting the fluid supply tube between the first and second handle portions, and wherein the first and second handle portions can be secured together to retain the fluid supply tube in the handle between the first and second handle portions.

22. The surgical device of claim 21, wherein the handle includes an interior channel partially defined by the first handle portion and partially defined by the second handle portion, and wherein the interior channel is configured to receive the fluid supply tube when the first handle portion and the second handle portion are secured together.

23. The surgical device of claim 22, wherein the interior channel is configured to retain the fluid supply tube by a friction fit.

24. The surgical device of claim 21, wherein the first handle portion and the second handle portion are connected to each other by a pivot connection, and wherein the first handle portion and the second handle portion can be partially separated from each other and secured together by pivoting the first handle portion and the second handle portion with respect to each other.

## Patentansprüche

1. Chirurgisches Instrument, das Folgendes aufweist: einen Griff; eine an dem Griff befestigte Klinge, und das **gekennzeichnet ist durch** mindestens eine Öffnung, die zum Sprühen eines Kryogens aus dem chirurgischen Instrument konfiguriert ist, wobei die mindestens eine Öffnung in oder auf mindestens einem des Griffs und der Klinge angeordnet ist.

2. Chirurgisches Instrument nach Anspruch 1, wobei die mindestens eine Öffnung eine Öffnung aufweist, die an einer der folgenden Stellen positioniert ist: einer Unterseite des Griffs, vor einer Spitze der Klinge, einer Seitenfläche der Klinge, und einer Oberkante der Klinge.

3. Chirurgisches Instrument nach Anspruch 1, das mindestens einen Kanal aufweist, der konfiguriert ist, um das Kryogen zu der mindestens einen Öffnung zu leiten, wobei der mindestens eine Kanal durch einen Innenraum des Griffs verläuft oder auf einer Außenwand des Griffs angeordnet ist.

4. Chirurgisches Instrument nach Anspruch 3, das einen ersten Fluidzuführschlauch aufweist, der konfiguriert ist, um in Fluidverbindung mit einer Quelle des Kryogens gebracht zu werden, wobei der mindestens eine Kanal konfiguriert ist, um in Fluidverbindung mit dem Fluidzuführschlauch gebracht zu werden.

5. Chirurgisches Instrument nach Anspruch 3, wobei die mindestens eine Öffnung in einem Fluidanlegeschlauch angeordnet ist, der an dem Griff oder der Klinge befestigt oder angeordnet ist, und wobei der mindestens eine Kanal einen Kanal aufweist, der durch den Fluidanlegeschlauch verläuft.

6. Chirurgisches Instrument nach Anspruch 3, wobei die mindestens eine Öffnung in einem Flüssigkeitsanlegeschlauch angeordnet ist, der an dem Griff oder der Klinge befestigt oder angeordnet ist, und wobei der mindestens eine Kanal einen Kanal aufweist, der durch den Flüssigkeitsanlegeschlauch verläuft.

7. Chirurgisches Instrument nach Anspruch 1 oder 3-6, wobei die mindestens eine Öffnung eingerichtet ist, um in Fluidverbindung mit einer Quelle des Kryogens zu stehen, und wobei die mindestens eine Öffnung konfiguriert ist, um das Kryogen auf ein Gewebe in der Nähe der Klinge zu sprühen oder ein Isotherm in der Nähe der Klinge zu erzeugen.

8. Chirurgisches Instrument nach Anspruch 3, wobei das Kryogen ein verflüssigtes Gas darstellt.

9. Chirurgisches Instrument nach Anspruch 3, wobei das Kryogen aus der Gruppe ausgewählt wird, die aus Sauerstoff, Stickstoff, Argon, Kohlendioxid und Luft besteht.

10. Chirurgisches Instrument nach Anspruch 1, das mindestens ein zum Erhitzen der Klinge konfiguriertes Heizelement aufweist.

11. Chirurgisches Instrument nach Anspruch 1, wobei die Klinge mindestens ein Kühlelement aufweist, wobei das mindestens eine Kühlelement optional mindestens einen Schlauch aufweist, der zum Tragen eines Kühlfluids eingerichtet ist, und wobei das Kühlfluid optional das Kryogen darstellt.

12. Chirurgisches Instrument nach Anspruch 1, wobei die Klinge entfernbar an dem Griff befestigt ist.

13. Chirurgisches Instrument nach Anspruch 1 oder 3, wobei das Instrument eine Absaugpassage aufweist, die zum Absaugen eines überschüssigen Anteils des Kryogens nach Sprühen des Kryogens konfiguriert ist.

14. Chirurgisches Instrument nach Anspruch 13, wobei die Absaugpassage koaxial mit dem Kanal ist oder an denselben angrenzt.

15. Chirurgisches Instrument nach Anspruch 13, das eine Absaughaube aufweist, die zum Leiten des überschüssigen Anteils des Kryogens in die Absaugpassage konfiguriert ist.

16. Chirurgisches Instrument nach Anspruch 14, wobei die Absaughaube um die mindestens eine Öffnung herum angeordnet ist.

17. Chirurgisches Instrument nach einem der Ansprüche 1, 2, 7-9 oder 10-12, das weiter Folgendes aufweist: einen Fluidzuführschlauch, der an dem Griff befestigt werden kann; wobei die mindestens eine Öffnung zum Sprühen des durch den Fluidzuführschlauch gelieferten Kryogens konfiguriert ist.

18. Chirurgisches Instrument nach Anspruch 16, das einen Kanal aufweist, der durch den Griff verläuft oder auf dem Griff angeordnet ist, wobei der Kanal konfiguriert ist, um den Fluidzuführschlauch festzuhalten.

19. Chirurgisches Instrument nach Anspruch 16, wobei der Fluidzuführschlauch konfiguriert ist, um in Fluidverbindung mit dem Kanal gebracht zu werden, und wobei die mindestens eine Öffnung an einem Ende des Kanals angeordnet ist.

20. Chirurgisches Instrument nach Anspruch 16, das einen Kanal aufweist, der durch den Griff verläuft oder an dem Griff angeordnet ist, wobei der Fluidzuführschlauch konfiguriert ist, um so durch den Kanal zu verlaufen, dass ein Ausgabeende des Fluidzuführschlauchs aus dem Griff durch ein Ende des Kanals vorsteht, und wobei die mindestens eine Öffnung eine Öffnung aufweist, die an dem Ausgabeende des Fluidzuführschlauchs angeordnet ist.

21. Chirurgisches Instrument nach Anspruch 16, wobei der Griff einen ersten Griffteil und einen zweiten Griffteil aufweist, wobei der erste und zweite Griffteil mindestens teilweise voneinander entlang einer axialen Richtung des Griffs zum Einsetzen des Fluidzuführschlauchs zwischen dem ersten und zweiten Griffteil getrennt werden können, und wobei der erste und zweite Griffteil aneinander befestigt werden können, um den Fluidzuführschlauch in dem Griff zwischen dem ersten und zweiten Griffteil festzuhalten.

22. Chirurgisches Instrument nach Anspruch 21, wobei der Griff einen Innenkanal enthält, der teilweise durch den ersten Griffteil begrenzt wird und teilweise durch den zweiten Griffteil begrenzt wird, und wobei der Innenkanal zum Aufnehmen des Fluidzuführschlauchs konfiguriert ist, wenn der erste Griffteil und der zweite Griffteil aneinander befestigt werden.

23. Chirurgisches Instrument nach Anspruch 22, wobei der Innenkanal konfiguriert ist, um den Fluidzuführschlauch durch Reibsitz festzuhalten.

24. Chirurgisches Instrument nach Anspruch 21, wobei der erste Griffteil und der zweite Griffteil miteinander durch eine Schwenkverbindung verbunden sind, und wobei der erste Griffteil und der zweite Griffteil durch Schwenken des ersten Griffteils und des zweiten Griffteils in Bezug zueinander teilweise voneinander getrennt und aneinander befestigt werden können.

## Revendications

1. Dispositif chirurgical, comportant : un manche ; une lame attachée au manche ; **caractérisé par** au moins un orifice configuré à des fins de pulvérisation d'un cryogène en provenance du dispositif chirurgical, ledit au moins un orifice étant disposé dans ou sur au moins l'un parmi le manche et la lame.

2. Dispositif chirurgical selon la revendication 1, dans lequel ledit au moins un orifice comporte un orifice positionné au niveau de l'un des emplacements suivants : une partie inférieure du manche, devant une pointe de la lame, une surface latérale de la lame, et un bord supérieur de la lame.

3. Dispositif chirurgical selon la revendication 1, comportant au moins un profilé en U configuré afin de diriger le cryogène jusqu'audit au moins un orifice, dans lequel ledit au moins un profilé en U s'étend au travers d'une partie intérieure du manche ou est disposé sur une paroi extérieure du manche.

4. Dispositif chirurgical selon la revendication 3, comportant un tube d'alimentation en fluide configuré pour être placé en communication fluidique avec une source du cryogène, dans lequel ledit au moins un profilé en U est configuré pour être placé en communication fluidique avec le tube d'alimentation en fluide.

5. Dispositif chirurgical selon la revendication 3, dans lequel ledit au moins un orifice est disposé dans un tube d'application de fluide attaché ou disposé sur le manche ou la lame, et dans lequel ledit au moins un profilé en U comporte un profilé en U s'étendant au travers du tube d'application de fluide.

6. Dispositif chirurgical selon la revendication 3, dans lequel ledit au moins un orifice est disposé dans un tube d'application de liquide attaché ou disposé sur le manche ou la lame, et dans lequel ledit au moins un profilé en U comporte un profilé en U s'étendant au travers du tube d'application de liquide.

7. Dispositif chirurgical selon la revendication 1 ou les revendications 3 à 6, dans lequel ledit au moins un orifice est adapté pour être en communication fluidique avec une source du cryogène, et dans lequel ledit au moins un orifice est configuré à des fins de pulvérisation du cryogène sur un tissu à proximité de la lame ou à des fins de création d'une isotherme à proximité de la lame.

8. Dispositif chirurgical selon la revendication 3, dans lequel le cryogène est un gaz liquéfié.

9. Dispositif chirurgical selon la revendication 3, dans lequel le cryogène est sélectionné dans le groupe constitué par de l'oxygène, de l'azote, de l'argon, du dioxyde de carbone, et de l'air.

10. Dispositif chirurgical selon la revendication 1, comportant au moins un élément de chauffage configuré afin de chauffer la lame.

11. Dispositif chirurgical selon la revendication 1, dans lequel la lame comporte au moins un élément de refroidissement, éventuellement dans lequel ledit au moins un élément de refroidissement comporte au moins un tube adapté pour porter un fluide de refroidissement, et éventuellement dans lequel le fluide de refroidissement est le cryogène.

12. Dispositif chirurgical selon la revendication 1, dans lequel la lame est attachée de manière amovible par rapport au manche.

13. Dispositif chirurgical selon la revendication 1 ou la revendication 3, dans lequel le dispositif comporte un passage d'aspiration configuré pour aspirer une partie excessive du cryogène après la pulvérisation du cryogène.

14. Dispositif chirurgical selon la revendication 13, dans lequel le passage d'aspiration est coaxial ou adjacent par rapport au profilé en U.

15. Dispositif chirurgical selon la revendication 13, comportant un capot d'aspiration configuré afin de diriger la partie excessive du cryogène jusqu'au passage d'aspiration.

16. Dispositif chirurgical selon la revendication 14, dans lequel le capot d'aspiration est disposé autour dudit au moins un orifice.

17. Dispositif chirurgical selon l'une quelconque des revendications 1, 2, 7 à 9 ou 10 à 12, comportant par ailleurs : un tube d'alimentation en fluide en mesure d'être attaché au manche ; dans lequel ledit au moins un orifice est configuré à des fins de pulvérisation du cryogène alimenté par le biais du tube d'alimentation en fluide.

18. Dispositif chirurgical selon la revendication 16, comportant un profilé en U s'étendant au travers du manche ou disposé sur le manche, dans lequel le profilé en U est configuré à des fins de retenue du tube d'alimentation en fluide.

19. Dispositif chirurgical selon la revendication 16, dans lequel le tube d'alimentation en fluide est configuré pour être placé en communication fluidique avec le profilé en U, et dans lequel ledit au moins un orifice est disposé au niveau d'une extrémité du profilé en U.

20. Dispositif chirurgical selon la revendication 16, comportant un profilé en U s'étendant au travers du manche ou disposé sur le manche, dans lequel le tube d'alimentation en fluide est configuré pour s'étendre au travers du profilé en U de telle sorte qu'une extrémité de distribution du tube d'alimentation en fluide fait saillie depuis le manche au travers d'une extrémité du profilé en U, et dans lequel ledit au moins un orifice comporte un orifice disposé au niveau de l'extrémité de distribution du tube d'alimentation en fluide.

21. Dispositif chirurgical selon la revendication 16, dans lequel le manche comporte une première partie de manche et une seconde partie de manche, dans lequel les première et seconde parties de manche sont au moins partiellement séparables l'une par rapport à l'autre dans une direction axiale du manche à des fins d'insertion du tube d'alimentation en fluide entre les première et seconde parties de manche, et dans lequel les première et seconde parties de manche peuvent être assujetties ensemble afin de retenir le tube d'alimentation en fluide dans le manche entre les première et seconde parties de manche.

22. Dispositif chirurgical selon la revendication 21, dans lequel le manche comprend un profilé en U intérieur partiellement défini par la première partie de manche et partiellement défini par la seconde partie de manche, et dans lequel le profilé en U intérieur est configuré pour recevoir le tube d'alimentation en fluide quand la première partie de manche et la seconde partie de manche sont assujetties ensemble.

23. Dispositif chirurgical selon la revendication 22, dans lequel le profilé en U intérieur est configuré à des fins de retenue du tube d'alimentation en fluide par une pose à pression.

24. Dispositif chirurgical selon la revendication 21, dans lequel la première partie de manche et la seconde partie de manche sont raccordées l'une par rapport à l'autre par une connexion à pivot, et dans lequel la première partie de manche et la seconde partie de manche peuvent être partiellement séparées l'une par rapport à l'autre et assujetties ensemble en faisant pivoter la première partie de manche et la seconde partie de manche l'une par rapport à l'autre.
